(19) Europäisches Patentamt
European Patent Office
Office européen des brevets

(11) Veröffentlichungsnummer: **0 545 170 A1**

# (12) EUROPÄISCHE PATENTANMELDUNG

(21) Anmeldenummer: **92119776.0**

(22) Anmeldetag: **20.11.92**

(51) Int. Cl.5: **C07D 215/14**, A61K 31/47

(30) Priorität: **03.12.91 DE 4139749**

(43) Veröffentlichungstag der Anmeldung:
**09.06.93 Patentblatt 93/23**

(84) Benannte Vertragsstaaten:
**AT BE CH DE DK ES FR GB GR IE IT LI LU MC NL PT SE**

(71) Anmelder: **BAYER AG**

**W-5090 Leverkusen 1 Bayerwerk(DE)**

(72) Erfinder: **Raddatz, Siegfried, Dr.**
**Jakob-Böhme-Strasse 21**
**W-5000 Köln 80(DE)**
Erfinder: **Mohrs, Klaus-Helmut, Dr.**
**Wildsteig 24**
**W-5600 Wuppertal 1(DE)**
Erfinder: **Matzke, Michael, Dr.**
**Am Ringelbusch 15**
**W-5600 Wuppertal 1(DE)**
Erfinder: **Fruchtmann, Romanis, Dr.**
**Weidenpescher Strasse 14**
**W-5000 Köln 60(DE)**
Erfinder: **Hatzelmann, Armin, Dr.**
**Alter Wall 3**
**W-7750 Konstanz(DE)**
Erfinder: **Müller-Pddinghaus, Reiner, Prof. Dr.**
**Klutstein 22a**
**W-5060 Bergisch Gladbach 2(DE)**

## (54) Chinolylmethoxyphenyl-essigsäureamide.

(57) Chinolylmethoxyphenyl-essigsäureamide werden hergestellt, indem man entweder entsprechende Carbonsäuren mit Aminen umsetzt oder entsprechende Cyanoverbindungen in Amide umwandelt und gegebenenfalls alkyliert. Die Chinolylmethoxyphenyl-essigsäureamide können als Wirkstoffe in Arzneimittel, insbesondere als Leukotriensynthesehemmer eingesetzt werden.

EP 0 545 170 A1

Die Erfindung betrifft Chinolylmethoxyphenyl-essigsäureamide, Verfahren zu ihrer Herstellung und ihre Verwendung in Arzneimitteln.

Es ist bereits bekannt, daß substituierte 4-(Chinolin-2-yl-methoxy)phenyl-essigsäure-Derivate und substituierte (Chinolin-2-yl-methoxy)phenyl-acylsulfonamide eine lipoxygenaseinhibierende Wirkung besitzen [vgl. EP 344 519 A und EP 399 291 A].

Die vorliegende Erfindung betrifft Chinolylmethoxyphenyl-essigsäureamide der allgemeinen Formel (I)

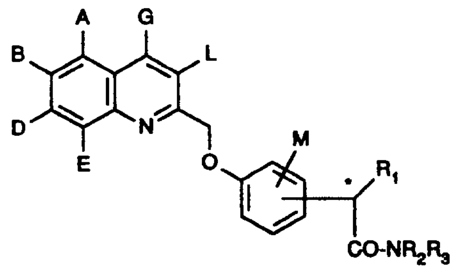

(I),

in welcher

A, B, D, E, G, L und M unabhängig voneinander
für Wasserstoff, Hydroxy, Halogen, Cyano, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,

$R^1$ für Cycloalkyl mit 3 bis 12 Kohlenstoffatomen steht, das gegebenenfalls durch geradkettiges oder verzweigtes Alkyl mit bis zu 8 Kohlenstoffatomen substituiert ist,

$R^2$ und $R^3$ gleich oder verschieden sind und
für Wasserstoff oder für geradkettiges oder verzweigtes Alkyl mit bis zu 8 Kohlenstoffatomen stehen, oder
für Benzyl stehen, oder
für Cycloalkyl mit 3 bis 12 Kohlenstoffatomen stehen, das gegebenenfalls durch geradkettiges oder verzweigtes Alkyl mit bis zu 8 Kohlenstoffatomen substituiert ist, oder

$R^2$ und $R^3$ zusammen mit dem Stickstoffatom einen heterocyclischen Ring der Formel

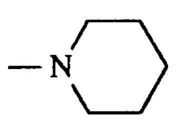

,

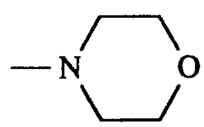

oder

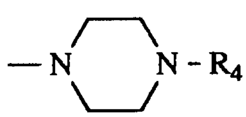

bilden, worin

$R^4$ geradkettiges oder verzweigtes Alkyl oder Alkenyl mit jeweils bis zu 8 Kohlenstoffatomen bedeutet, die gegegebenenfalls durch Phenyl substituiert sind oder Phenyl bedeutet, das gegebenenfalls durch Halogen oder Trifluormethyl substituiert ist,

gegebenenfalls in einer isomeren Form und deren Salze.

Überraschenderweise zeigen die erfindungsgemäßen Chinolylmethoxyphenyl-essigsäureamide der allgemeinen Formel (I) eine hohe in vitro Aktivität als Leukotriensynthesehemmer.

Im Rahmen der vorliegenden Erfindung werden physiologisch unbedenkliche Salze bevorzugt. Physiologisch unbedenkliche Salze der Chinolylmethoxyphenyl-essigsäureamide können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Die erfindungsgemäßen Verbindungen existieren in stereoisomeren Formen (*), die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lasseri sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher

A, B, D, E, G, L und M — unabhängig voneinander für Wasserstoff, Hydroxy, Fluor, Chlor, Brom, Carboxy, Nitro, Trifluormethyl, Trifluormethoxy oder für geradkettiges oder verzweigtes Alkyl oder Alkoxy mit jeweils bis zu 6 Kohlenstoffatomen stehen, oder für Phenyl stehen, das gegebenenfalls durch Fluor, Chlor, Brom, Hydroxy, Nitro oder Cyano substituiert ist,

$R^1$ — für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 6 Kohlenstoffatomen substituiert sind,

$R^2$ und $R^3$ — gleich oder verschieden sind und für Wasserstoff oder für geradkettiges oder verzweigtes Alkyl mit bis zu 6 Kohlenstoffatomen stehen, oder für Benzyl stehen, oder für Cyclopropyl, Cyclobutyl, Cyclopentyl, Cyclohexyl, Cycloheptyl oder Cyclooctyl stehen, die gegebenenfalls durch geradkettiges oder verzweigtes Alkyl mit bis zu 6 Kohlenstoffatomen substituiert sind, oder

$R^2$ und $R^3$ — zusammen mit dem Stickstoffatom einen heterocyclischen Ring der Formel

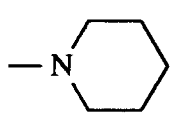

oder

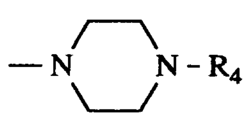

bilden, worin

$R^4$ — geradkettiges oder verzweigtes Alkyl oder Alkenyl mit jeweils bis zu 6 Kohlenstoffatomen bedeutet, die gegebenenfalls durch Phenyl substituiert sind oder Phenyl bedeutet, das gegebenenfalls durch Fluor, Chlor oder Trifluormethyl substituiert ist,

gegebenenfalls in einer isomeren Form und deren Salze.

Besonders bevorzugt sind Verbindungen der allgemeinen Formel (I),
in welcher

A, B, D, E, G, L und M unabhängig voneinander
für Wasserstoff, Hydroxy, Fluor, Chlor, Brom oder geradkettiges oder verzweigtes Alkyl mit bis zu 4 Kohlenstoffatomen stehen,

$R^1$ für Cyclopropyl, Cyclopentyl, Cyclohexyl oder Cycloheptyl steht, die gegebenenfalls durch geradkettiges oder verzweigtes Alkyl mit bis zu 4 Kohlenstoffatomen substituiert sind,

$R^2$ und $R^3$ gleich oder verschieden sind und
für Wasserstoff oder für geradkettiges oder verzweigtes Alkyl mit bis zu 4 Kohlenstoffatomen stehen, oder
für Benzyl stehen, oder
für Cyclopropyl, Cyclopentyl, Cyclohexyl oder Cycloheptyl stehen, die gegebenenfalls durch geradkettiges oder verzweigtes Alkyl mit bis zu 4 Kohlenstoffatomen substituiert sind, oder

$R^2$ und $R^3$ zusammen mit dem Stickstoffatom einen heterocyclischen Ring der Formel

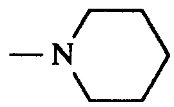

oder

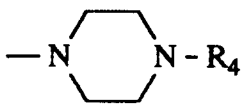

bilden, worin

$R^4$ geradkettiges oder verzweigtes Alkyl oder Alkenyl mit jeweils bis zu 4 Kohlenstoffatomen bedeutet, die gegebenenfalls durch Phenyl substituiert sind
oder Phenyl bedeutet, das gegebenenfalls durch Fluor, Chlor oder Trifluormethyl substituiert ist,

gegebenenfalls in einer isomeren Form und deren Salze.

Ganz besonders bevorzugt sind Verbindungen der allgemeinen Formel (I), in welcher
A, B, D, E, G und L für Wasserstoff oder Chlor stehen.

Ebenso sind solche ganz besonders bevorzugt, in denen der Rest

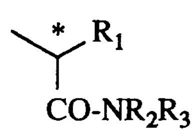

in 4-Stellung zum Chinolylmethoxyrest steht.

Außerdem wurden Verfahren zur Herstellung der erfindungsgemäßen Verbindungen der allgemeinen Formel (I) gefunden, dadurch gekennzeichnet, daß man

[A] Säuren der allgemeinen Formel (II)

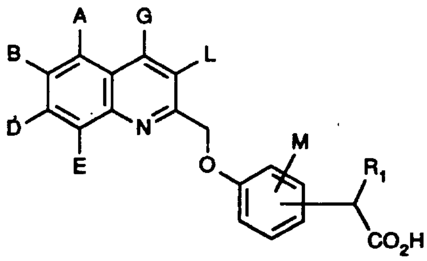

(II),

in welcher
A, B, D, E, G, L, M und $R^1$ die oben angegebene Bedeutung haben,
nach Aktivierung der Carbonsäurefunktion, beispielsweise über die Säurehalogenide-, Anhydride oder Imidazolide, in organischen Lösemitteln mit Aminen der allgemeinen Formel (III)

$HNR^{2'}R^{3'}$ (III),

in welcher
$R^{2'}$ und $R^{3'}$ die oben angegebene Bedeutung von $R^2$ und $R^3$ haben, aber nicht gleichzeitig für Wasserstoff stehen.
in inerten Lösemitteln, gegebenenfalls in Anwesenheit einer Base amidiert, oder mit Ammoniak/Ammoniumchlorid umsetzt, oder
[B] Cyano-Verbindungen der allgemeinen Formel (IV)

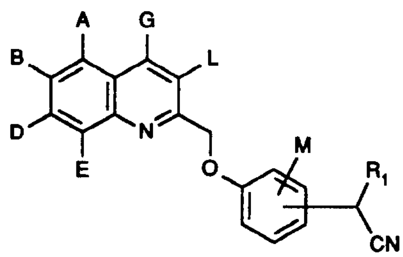

(IV),

in welcher
A, B, D, E, G, L, M und $R^1$ die oben angegebene Bedeutung haben,
mit Säuren, vorzugsweise Salzsäure, umsetzt,
und im Fall, daß $R^2$ und/oder $R^3$ nicht Wasserstoff bedeuten,
gegebenenfalls auch eine Alkylierung der Aminogruppe anschließt,
und gegebenenfalls die Substituenten A, B, D, E, G, L und M nach üblichen Methoden variiert.

Das erfindungsgemäße Verfahren kann durch folgendes Formelschema beispielhaft erläutert werden:

[A]

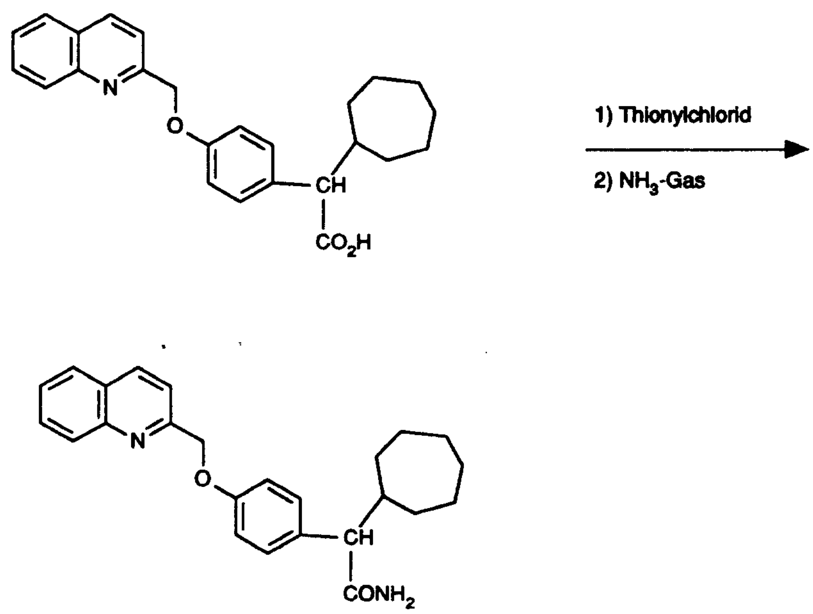


[B]

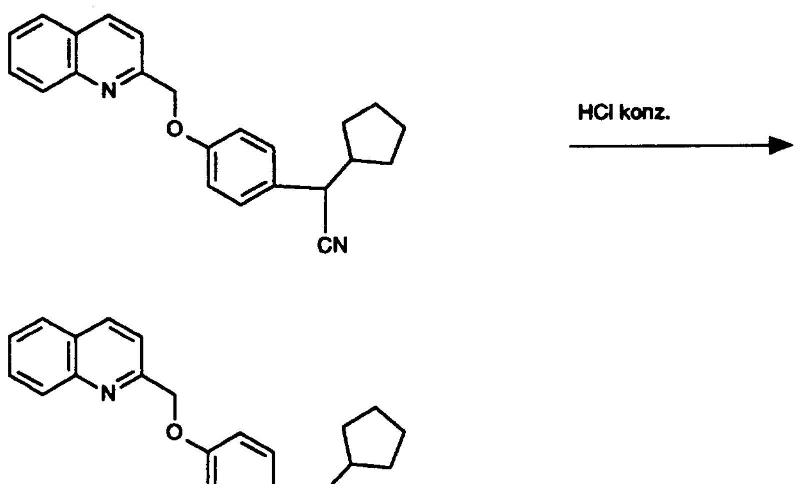


Als Lösemittel eignen sich hierbei inerte organische Lösemittel, die sich unter den Reaktionsbedingungen nicht verändern. Hierzu gehören Ether, wie Diethylether oder Teträhydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton und Dimethylformamid.

Als Basen für die einzelnen Verfahrensschritte, insbesondere für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.butylat, oder organische Amine wie Benzyltrimethylammoniumhydroxid, Tetrabutylammoniumhydroxid, Pyridin, Triethylamin, Piperidin oder N-Methylpiperidin. Bevorzugt sind Kaliumcarbonat, Natriumhydrid, Natriumhydrogencarbonat und Piperidin.

Die Amidierung wird im allgemeinen in einem Temperaturbereich von 0 °C bis 150 °C, bevorzugt bei 25 °C bis 40 °C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Insbesondere wenn $R^2$,$R^3$ die Bedeutung von Wasserstoff haben, hat es sich als zweckmäßig erwiesen die Umsetzung im Ammoniakstrom, unter Umständen mit leichtem Überdruck, durchzuführen.

Zur Aktivierung der Carbonsäure eignen sich die üblichen Reagenzien wie anorganische Halogenide, beispielsweise Thionylchlorid, Mesylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Säuren für die Überführung der CN-Gruppe in den Verbindungen der allgemeinen Formel (IV) in die Amidgruppe eignen sich beispielsweise Salzsäure, Salzsäure/Wasser, $BF_3$/Essigsäure, Salzsäure/Ameisensäure oder Wasserstoffperoxid/Natronlauge/Ethanol/Wasser. Bevorzugt ist Salzsäure.

Die reinen Enantiomeren der erfindungsgemäßen Verbindungen der allgemeinen Formel (I) können beispielsweise hergestellt werden, indem man die entsprechenden unsubstituierten enantiomerenreinen Säuren nach üblicher Methode trennt und anschließend wie oben aufgeführt weiter umsetzt.

Die Säuren der allgemeinen Formel (II) sind an sich bekannt (M = H) oder können nach üblicher Methode hergestellt werden [vgl. DOS 38 18 443]. Im Fall, daß M nicht Wasserstoff bedeutet, sind die Verbindungen als konkrete Stoffvertreter teilweise neu und können durch Verseifung nach üblicher Methode aus den entsprechenden Estern hergestellt werden.

Die Amine der allgemeinen Formel (III) sind ebenfalls bekannt oder können nach üblichen Methoden hergestellt werden.

Die Verbindungen der allgemeinen Formel (IV) sind mit der oben angegebenen Bedeutung von $R^1$ neu und können hergestellt werden, indem man Cyano-Verbindungen der allgemeinen Formel (V)

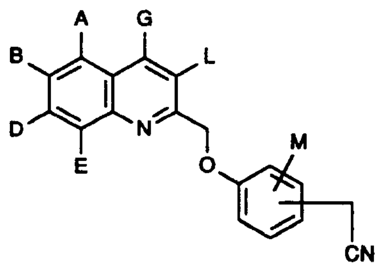

(V),

in welcher

A, B, D, E, G, L und M die oben angegebene Bedeutung haben,

mit Verbindungen der allgemeinen Formel (VI)

$R^1$-Z (VI),

in welcher

$R^1$ die oben angegebene Bedeutung hat und

Z für Halogen, vorzugsweise für Chlor oder Brom steht,

in einem der obengenannten Lösemitteln, vorzugsweise Dimethylformamid, in Anwesenheit einer Base, vorzugsweise Natriumhydroxid, alkyliert.

Die Verbindungen der Formel (V) sind als Zwischenprodukte teilweise aus der PCT WO 87/05510 bekannt.

Ebenso sind die Verbindungen der allgemeinen Formel (VI) bekannt oder können nach üblicher Methode hergestellt werden (vgl. z.B. Beilstein 5,19).

Die erfindungsgemäßen Verbindungen können als Wirkstoffe in Arzneimitteln eingesetzt werden. Die Stoffe können als Hemmer von enzymatischen Reaktionen im Rahmen des Arachidonsäurestoffwechsels, insbesondere der 5-Lipoxygenase, wirken.

Die Verbindungen der allgemeinen Formel (I) zeigen überraschenderweise eine hohe in vitro Aktivität als Leukotriensynthesehemmer und eine starke in vivo Wirkung nach oraler Applikation.

Sie sind somit bevorzugt zur Behandlung und Verhütung von Entzündungen, insbesondere von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen Verbindungen können sowohl in der Humanmedizin als auch in der Veterinärmedizin angewendet werden.

Die pharmakologischen Wirkdaten der erfindungsgemäßen Substanzen werden durch folgende Methode bestimmt:
Als Maß für die 5-Lipoxygenase-Hemmung in vitro wurde die Freisetzung von Leukotrien $B_4$ ($LTB_4$) an polymorphkernigen Humanleukozyten (PMN) nach Zugabe von Substanzen und Ca-Ionophor mittels reverse phase HPLC nach Borgeat, P. et al., Proc. Nat. Acad. Sci. 76, 2148-2152 (1979), bestimmt.

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-%, der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Ausgangsverbindungen

Beispiel I

4-(Chinolin-2-yl-methoxy)phenyl-acetonitril

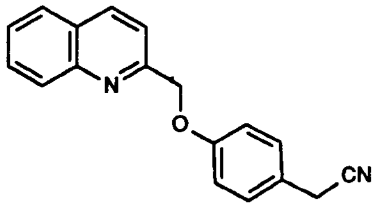

18 g (0,101 mol) Chinolin-2-yl-methylchlorid, 13,3 g (0,1 mol) 4-Hydroxyphenylacetonitril und 14 g (0,1 mol) Kaliumcarbonat (pulverisiert und getrocknet bei 110 °C) werden in 400 ml trockenem Aceton 72 Stunden zum Sieden erhitzt. Anschließend läßt man erkalten, filtriert vom Festprodukt ab und dampft das Filtrat i.V. zur Trockene ein. Man nimmt den Rückstand in 250 ml Dichlormethan auf, wäscht zweimal mit 250 ml 2 n Natronlauge, dann mit Wasser neutral, trocknet mit Natriumsulfat und dampft i.V. zur Trockene ein. Die Rekristallisation erfolgt aus Diisopropylether/Essigester.
Ausbeute: 21,6 g (78,8% der Theorie)
Fp.: 104 - 105 °C (farblose Kristalle)

Beispiel II

2-[4-(Chinolin-2-yl-methoxy)phenyl]-2-cyclopentyl-acetonitril

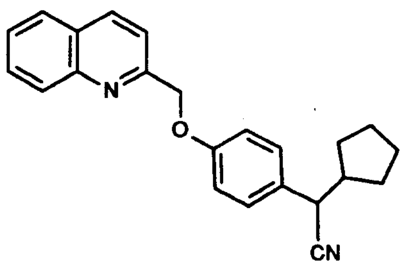

Man suspendiert bei 0 °C 0,6 g (80%iges) Natriumhydrid (0,02 mol) in 40 ml abs. DMF und tropft 5,5 g (0,02 mol) der Verbindung aus Beispiel I in 20 ml DMF zu. Es setzt eine Wasserstoffentwicklung ein. Die Temperatur steigt dabei auf Raumtemperatur an. Man läßt noch 1 Stunde bei Raumtemperatur nachrühren, kühlt dann auf 0 °C ab und tropft 3 g (0,02 mol) Cyclopentylbromid zu. Man läßt noch über Nacht reagieren, engt dann im Vakuum zur Trockene ein und rührt den Rückstand mit 180 ml Wasser / Dichlormethan (1:1) aus. Die organische Phase wird abgetrennt, getrocknet, auf ein kleines Volumen eingeengt und säulenchromatographisch getrennt (Kieselgel 60, Laufmittel: Toluol/Essigester = 9:1).
$R_f = 0,5$
Ausbeute: 4 g (53% der Theorie)
Fp.: 87 °C (farblose Kristalle)

Beispiel III

2-[3-Isobutyl-4-(chinolin-2-yl-methoxy)phenyl]-2-cyclohexyl-essigsäuremethylester

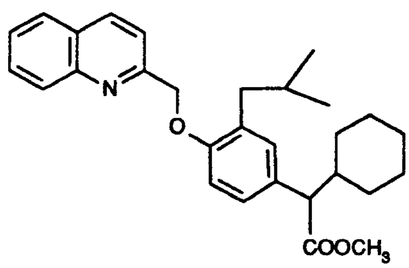

Unter Argonatmosphäre werden 12 g (0,033 mol) 2-[3-Isobutyl-4-(chinolin-2-yl-methoxy)-phenylessigsäuremethylester und 6,52 g (0,04 mol) ≙ 4,0 ml Cyclohexylbromid in 36 ml DMF gelöst und auf 0 °C abgekühlt. Unter Rühren tropfte man dazu 4,88 g (0,04 ml) Kaliumtertiärbutylat, gelöst in 80 ml DMF. Nach ca. 2 h Reaktionszeit läßt man die Temperatur auf Raumtemperatur ansteigen, säuert mit 2 n Salzsäure an und engt i.V. zur Trockne ein. Man rührt den verbleibenden Rest mit 100 ml Dichlormethan und 50 ml Wasser aus, trennt die organische Phase ab, trocknet sie mit $Na_2SO_4$, engt i.V. auf ein kleines Volumen ein und trennt den verbleibenden Rest säulenchromatographisch (Kieselgel 60, Laufmittel:Dichlormethan/Essigester = 100/2).
leicht gelbliches Öl, Ausbeute: 13 g (88,4 % d.Th.)

Beispiel IV

2-[3-Isobutyl-4-(chinolin-2-yl-methoxy)phenyl]-2-cyclohexyl-essigsäure

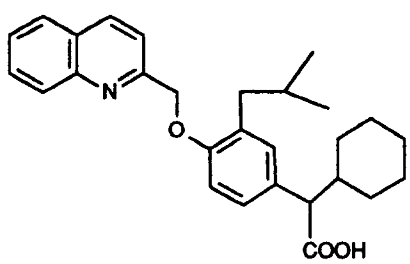

10,2 g (0,0236 mol) der Verbindung aus Beispiel III werden in 70 ml 2-Propanol aufgenommen und mit 50 ml 1 n Natronlauge über Nacht zum Sieden erhitzt. Nach dem Erkalten neutralisiert man mit 50 ml 1 n Salzsäure. Den angefallenen, farblosen Niederschlag saugt man ab, wäscht und trocknet ihn und rekristallisiert anschließend aus Diisopropylether.
farbl. Kristalle: Fp.: 130 °C Ausbeute: 9,5 g (96,3 % d.Th.)

Beispiele V und VI

(+)-2-[3-Isobutyl-4-(chinolin-2-yl-methoxy)phenyl]-2-cyclohexyl-essigsäure (V)
(-)-2-[3-Isobutyl-4-(chinolin-2-yl-methoxy)phenyl]-2-cyclohexyl-essigsäure (VI)

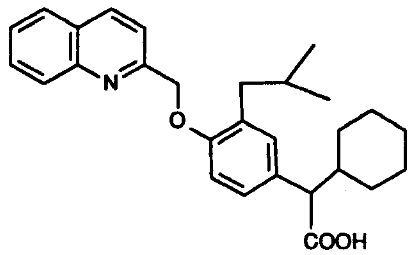

Die Titelverbindungen werden durch Trennung des Racemates (Beispiel IV) durch chromatographische Trennung an chiralen Säulen erhalten
(+)-Enantiomer: spez. Drehung: 17,96 $CHCl_3$ (V) mol. Drehung: 77,41
(-)-Enantiomer: spez. Drehung: -18,86 $CHCl_3$ (VI) mol. Drehung: -81,28

Beispiel VII

2-[3-Isobutyl-4-(chinolin-2-yl-methoxy)phenyl]-2-cycloheptyl-essigsäuremethylester

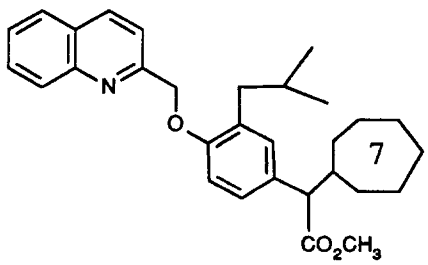

10 g (0,0275 mol) 2-[3-Isobutyl-4-(chinolin-2-yl-methoxy)-phenylessigsäuremethylester werden in Analogie zur Vorschrift des Beispiels III mit 10,04 g (0,055 mol) Cycloheptylbromid und 6,17 g (0,055 mol) Kaliumtertiärbutylat zur Titelverbindung umgesetzt.

Beispiel VIII

2-[3-Isobutyl-4-(chinolin-2-yl-methoxy)phenyl]-2-cycloheptyl-essigsäure

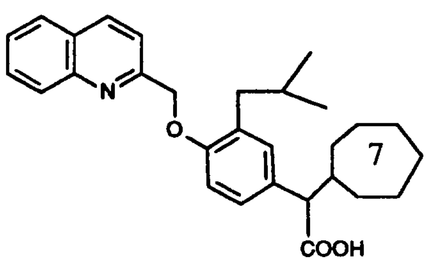

In Analogie zur Vorschrift des Beispiels IV wird die Titelverbindung aus der obigen Verbindung in 50 ml 1n Natronlauge mit anschließendem Ansäuern hergestellt.
farbl. Kristalle: Fp.: 112 °C
Ausbeute: 11,3g

Beispiele IX und X

(+)-2-[3-Isobutyl-4-(chinolin-2-yl-methoxy)phenyl]-2-cycloheptyl-essigsäure (IX)
(-)-2-[3-Isobutyl-4-(chinolin-2-yl-methoxy)phenyl]-2-cycloheptyl-essigsäure (X)

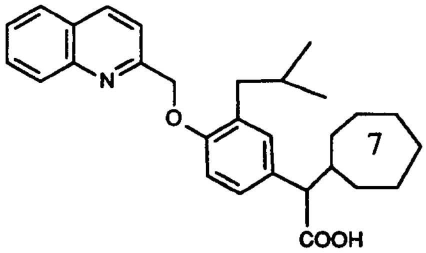

(+)-Enantiomer: spez. Drehung: 15,72, Lösungm. $CHCl_3$, mol. Drehung: 69,96, (Beispiel IX)
(-)-Enantiomer: spez. Drehung: -18,7, Lösungsm. $CHCl_3$ mol. Drehung: -86,19 (Beispiel X)
Die Titelverbindungen werden aus dem Racemat (Beispiel VIII) durch chromatographische Trennung an chiralen Säulen erhalten.

Beispiel XI

2-[3-Isobutyl-4-(chinolin-2-yl-methoxy)phenyl]-2-cyclopentyl-essigsäuremethylester

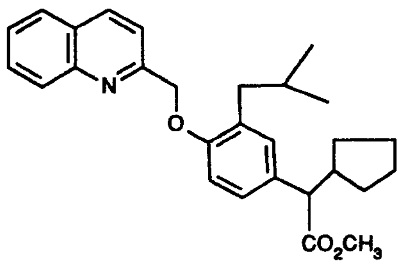

In Analogie zu den Vorschriften der Beispiele III und VII wird die Titelverbindung aus 10 g (0,0275 mol) 2-[3-Isobutyl-4-(chinolin-2-yl-methoxy)phenyl-essigsäuremethylester, 8,2 g (0,55 mol) Cyclopentylbromid und 6,17 g (0,055 mol) Kaliumtertiärbutylat hergestellt.
gelbbraunes Öl
Ausbeute: quantitativ

Beispiel XII

2-[3-Isobutyl-4-(chinolin-2-yl-methoxy)phenyl]-2-cyclopentyl-essigsäure

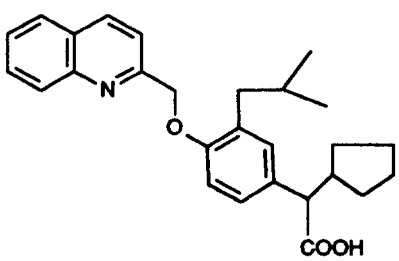

In Analogie zu den Vorschriften der Beispiele IV und VIII wird die Titelverbindung aus der Verbindung des Beispiels XI durch Verseifung mit 30 ml Natronlauge und anschließendem Ansäuern hergestellt.
leicht gelbliche Kristalle, Fp.: 120 °C
Ausbeute: 10,5g (91,5 % d.Th.)

Beispiele XIII und XIV

(+)-2-[3-Isobutyl-4-(chinolin-2-yl-methoxy)phenyl]-2-cyclopentyl-essigsäure (XIII)
(-)-2-[3-Isobutyl-4-(chinolin-2-yl-methoxy)phenyl]-2-cyclopentyl-essigsäure (XIV)

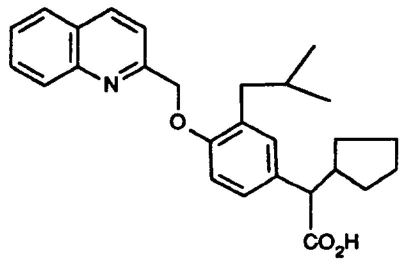

Die Titelverbindungen werden durch chromatographische Trennung der Verbindung des Beispiels XII an chiralen Säulen hergestellt.
(+)-Enantiomer: spez. Drehung: 44,56, (THF) mol. Drehung: 185,84 (Beispiel XIII)
(-)-Enantiomer: spez. Drehung: - 41,07, (THF) mol. Drehung: -171,28 (Beispiel IV)

Herstellungsbeispiele

Beispiel 1

2-[4-(Chinolin-2-yl-methoxy)phenyl]-2-cyclopentyl-essigsäureamid

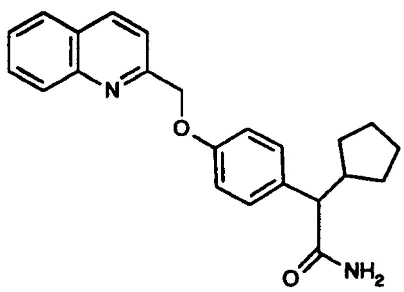

2 g (5,8 mmol) der Verbindung aus Beispiel II wurden in 6 ml konz. Salzsäure suspendiert und über Nacht bei 40 °C gerührt. Nach dem Abkühlen auf Raumtemperatur wird bis zur völligen Lösung THF zugesetzt und mit Natriumhydrogencarbonatlösung neutralisiert. Die organische Phase wird abgetrennt, mit Natriumsulfat getrocknet und im Vakuum auf ein kleines Volumen eingeengt. Die Trennung erfolgt säulenchromatographisch (Kieselgel 60, Laufmittel: Dichlormethan/Essigester Eisessig = 80/15/15).
$R_f$ = 0,35 (bei $R_f$ ca. 0.68 liegt die Säure)
Ausbeute: 1,28 g (71,3% der Theorie)
Fp.: 178 °C (farblose Kristalle)

Beispiele 2 und 3

(+)-2-[4-(Chinolin-2-yl-methoxy)phenyl]-2-cyclopentyl-essigsäureamid (2)
(-)-2-[4-(Chinolin-2-yl-methoxy)phenyl]-2-cyclopentyl-essigsäureamid (3)

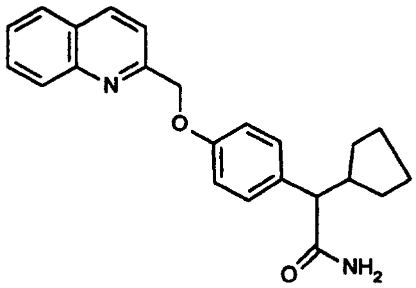

Die Beispiele (2) und (3) werden in Analogie zur Vorschrift des Beispiels 1 aus den enantiomerenreinen Säuren hergestellt.
$[\alpha]_D$ = + 30,5 (THF) Beispiel (2)
$[\alpha]_D$ = - 40,2 (THF) Beispiel (3).

Beispiel 4

2-[4-(Chinolin-2-yl-methoxy)phenyl]-2-cycloheptyl-essigsäureamid

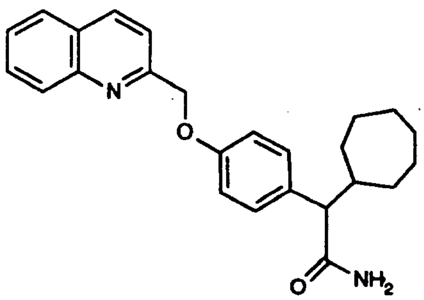

10 g (0,0257 mol) 2-[4-(Chinolin-2-yl-methoxy)phenyl]-2-cycloheptyl-essigsäure werden unter Argon in 100 ml getrocknetem Dichlormethan suspendiert, tropfenweise mit 8,3 g (5,1 ml) (0,07 mol) Thionylchlorid (frisch destilliert) versetzt und unter Rückfluß 2 Stunden erhitzt. Man dampft anschließend im Vakuum auf ein kleines Volumen ein, nimmt schnell in 20 ml getrocknetem Dichlormethan auf, kühlt auf -20 °C ab und leitet bis zur Sättigung einen trockenen Ammoniakstrom durch die Lösung. Es fällt ein farbloser Niederschlag an. Man läßt noch über Nacht bei Raumtemperatur nachrühren, rührt die Suspension mit Wasser aus, filtriert, wäscht mit Wasser nach und rekristallisiert den Filterkuchen aus THF.
Ausbeute: 9,7 g (97% der Theorie)
Fp.: 178 °C (farblose Kristalle)

Beispiele 5 und 6

(+)-2-[4-(Chinolin-2-yl-methoxy)phenyl]-2-cycloheptyl-essigsäureamid (5)
(-)-2-[4-(Chinolin-2-yl-methoxy)phenyl]-2-cycloheptyl-essigsäureamid (6)

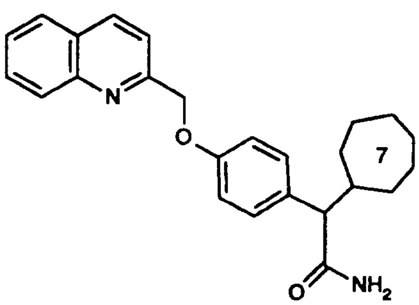

Die Verbindungen (5) und (6) werden in Analogie zur Vorschrift des Beispiels 4 aus den enantiomerenreinen Säuren hergestellt.
$[\alpha]_D$ = + 23,5 (THF) Beispiel (5)
$[\alpha]_D$ = - 21,9 (THF) Beispiel (6).

Beispiel 7

N-Methyl-2-[4-(Chinolin-2-yl-methoxy)phenyl]-2-cycloheptyl-essigsäureamid

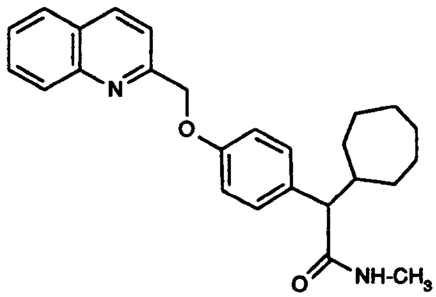

6 g 2-[4-(Chinolin-2-yl-methoxy)phenyl]-2-cycloheptylessigsäure (0,015 mol) und 2,67 g (0,0165 mol) Carbonyldiimidazol werden in 60 ml THF gelöst. In diese Lösung leitet man bei 40 °C einen getrockneten (NaOH-Plättchen) Methylaminstrom ein. Anschließend engt man i.V. zur Trockene ein, rührt mit 50 ml Wasser und 50 ml Toluol aus, trennt die organischen Phasen ab, trocknet mit Natriumsulfat, engt auf ein kleines Volumen ein und trennt das Gemisch säulenchromatographisch (Kieselgel 60, Laufmittel: Toluol/Essigester/Eisessig = 7/2/1). Die isolierte Probe wird aus Diisopropylether rekristallisiert.
Ausbeute: 3,66 g (58,8% der Theorie)
Fp.: 131 °C (farblose Kristalle)

Beispiel 8

2-[4-(Chinolin-2-yl-methoxy)phenyl]-2-cycloheptyl-essigsäurepiperidid

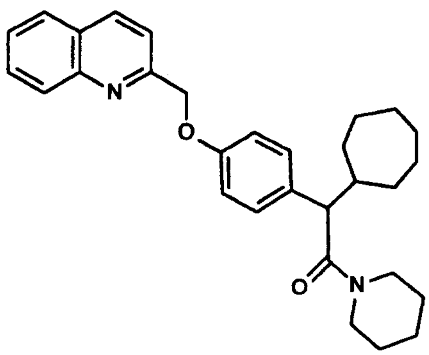

Analog Beispiel 7 wird die Titelverbindung aus 6 g (0,015 mol) 2-[4-(Chinolin-2-yl-methoxy)phenyl]-2-cycloheptyl-essigsäure, 5,34 g (0,033 mol) Carbonyldiimidazol und 170 g (2 mol) Piperidin erhalten. Die chromatographische Trennung erfolgt mit Kieselgel 60, Laufmittel: Dichlormethan/Methanol = 100/5.
Ausbeute: 0,8 g (11,7% der Theorie)
Fp.: 125 °C (farblose Kristalle)
In Analogie zu den oben aufgeführten Herstellungsvorschriften können die in Tabellen 1, 2 und 3 aufgeführten Verbindungen hergestellt werden:

Tabelle 1:

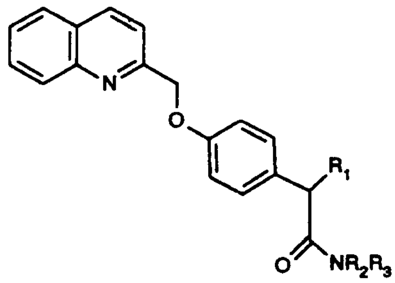

| Bsp.-Nr. | $R^1$ | $R^2$ | $R^3$ | Ausbeute (% d.Th.) | Fp. (°C) |
|---|---|---|---|---|---|
| 9 | 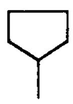 | $-C_2H_5$ | $-C_2H_5$ | | |
| 10 | 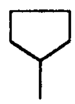 | 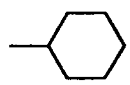 | H | | |
| 11 | 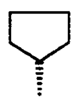 6 | $-CH_2$ | H | 75 | 138 |
| 12 | 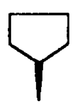 5 | $-CH_2$ | H | 73 | 137 |
| 13 | 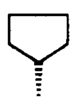 5 | $-C_2H_5$ | H | 63 | 145 |
| 14 | 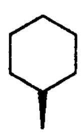 | $-C_2H_5$ | H | 75 | 144 |

Tabelle 2:

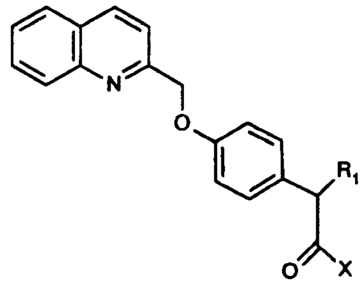

| Bsp.-Nr. | $R^1$ | X | Salz | Enantiomer |
|---|---|---|---|---|
| 15 | 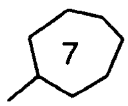 | 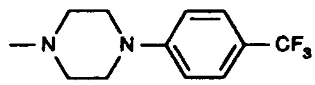 | | |
| 16 | 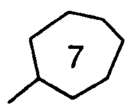 | 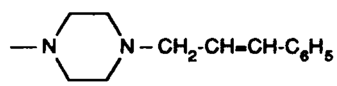 | | |
| 17 | 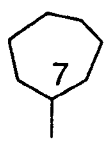 | 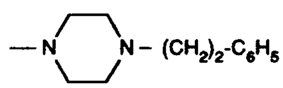 | HCl | |
| 18 | 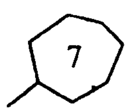 | 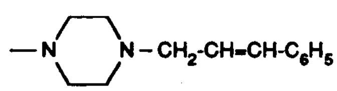 | | (+) |
| 19 | 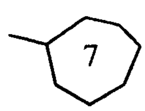 | 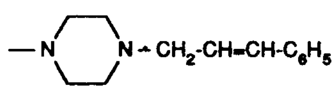 | | (-) |

Tabelle 3:

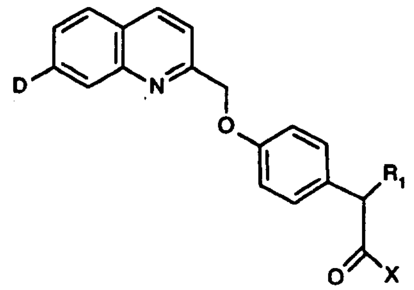

| Bsp.-Nr. | D | $R^1$ | X |
|---|---|---|---|
| 20 | Cl | 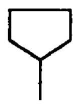 | 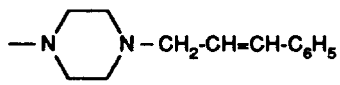 |

Beispiel 21

2-[3-Isobutyl-4-(chinolin-2-yl-methoxy)-phenyl]-2-cyclohexylacetamid

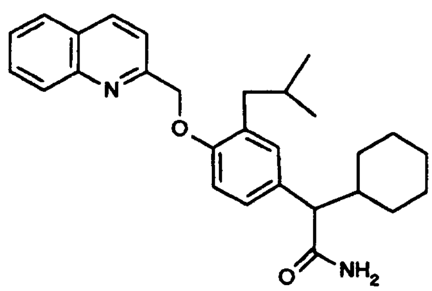

1,8 g (0,004 mol) 2-[3-Isobutyl-4-(chinolin-2-yl-methoxy)-phenyl]-2-cyclohexylessigsäure und 9,65 g (0,004 mol) N,N'-Carbonyl-diimidazol werden in 30 ml abs. THF gelöst und über Nacht bei 50 °C gerührt. Man dampft im Vakuum zur Trockne ein, nimmt in 50 ml Essigester auf und schüttelt zweimal mit 50 ml Wasser aus. Die organische Phase wird abgetrennt, mit $Na_2SO_4$ getrocknet und im Vakuum zur Trockne eingedampft. Rekristallisation erfolgt am Diisopropylether.
Ausbeute: 1,45 g (76,3 % der Theorie) farblose Kristalle.

Man löst 1,55 g (0,003 mol) des so hergestellten Imidazolids in 20 ml absolutem THF, versetzt mit einer Spatelspitze Ammoniumchlorid und leitet bei 50° einen trockenen Ammoniakstrom ein (ca. 5 h). Die gesättigte Mischung läßt man über Nacht stehen. Es fällt ein farbloser Niederschlag an. Man dampft alles zur Trockne ein und rekristallisiert den Rückstand aus Diisopropylether.
Ausbeute: 0,7 g (54,3% der Theorie) farblose Kristalle; Fp.: 193-5 °C.

Beispiel 22

N,N-Dimethyl-2-[3-isobutyl-4-(chinolin-2-yl-methoxy)-phenyl]-2-cycloheptylacetamid

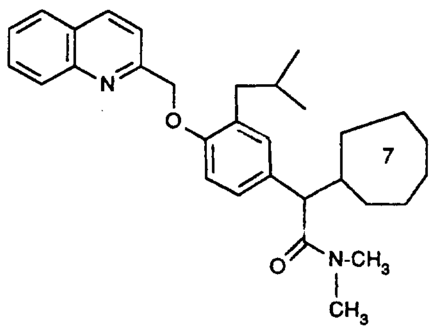

Die Titelverbindung wird in Ananlogie zur Vorschrift des Beispiels 21 aus 4,0 g (0,0091 mol) 2-[2-Isobutyl-4-(chinolin-2-yl-methoxy)phenyl]-2-cycloheptylessigsäure (Beispiel VIII), 1,8 g (0,0111 mol) N,N'-Carbonyldiimidazol und Dimethylamingas hergestellt. Die Trennung des Reaktionsgemisches erfolgt säulenchromatographisch (Kieselgel 60, Toluol/Essigester/Eisessig = 8/1/1; $R_f$ = 0,4).
Ausbeute: 0,8 g (21 % der Theorie) farblose Kristalle; Fp.: 114 °C.

Beispiel 23

2-[3-Isobutyl-4-(chinolin-2-yl-methoxy)phenyl]-2-cycloheptylacetamid

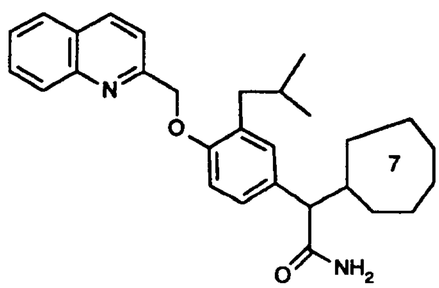

Die Titelverbindung wird in Analogie zur Vorschrift des Beispiels 21 aus 4,6 g (0,0104 mol) 2-[3-Isobutyl-4-(chinolin-2-yl-methoxy)phenyl]-2-cycloheptylessigsäure (Beispiel VIII), 2,2 g (0,013 mol) N,N'-Carbonyldiimidazol und Ammoniak hergestellt. Rekristallisation erfolgt aus Toluol; farblose Kristalle, Fp.: 193 °C.
Ausbeute: 2,9 g (63 % der Theorie).

Beispiel 24

N-Methyl-2-[3-isobutyl-4-(chinolin-2-yl-methoxy)-phenyl]-2-cycloheptylacetamid

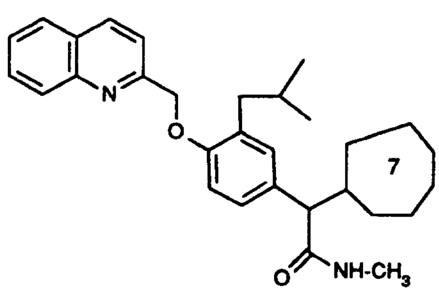

In Analogie zur Vorschrift des Beispiels 21 wird die Titelverbindung aus 3,0 g (0,00674 mol) 2-[3-Isobutyl-4-(chinolin-2-yl-methoxy)phenyl]-2-cycloheptylessigsäure, 1,4 g (0,0082 mol) N,N'-Carbonyldiimidazol und Methylamingas (50-60 °C, 5 h) hergestellt. Die Rekristallisation erfolgt aus Diisopropylether; farblose Kristalle, Fp.: 141 °C.

Ausbeute: 2,5 g (81 % der Theorie).

**Patentansprüche**

1. Chinolylmethoxyphenyl-essigsäureamide der allgemeinen Formel

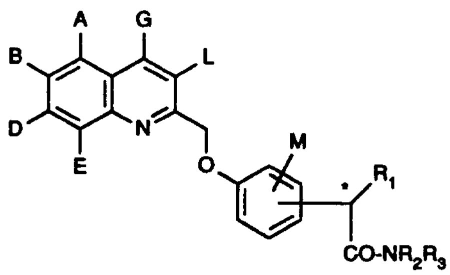

(I),

in welcher

A, B, D, E, G, L und M unabhängig voneinander für Wasserstoff, Hydroxy, Halogen, Cyano, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder für Aryl mit 6 bis 10 Kohlenstoffatomen stehen, das gegebenenfalls durch Halogen, Hydroxy, Nitro oder Cyano substituiert ist,

$R^1$ für Cycloalkyl mit 3 bis 12 Kohlenstoffatomen steht, das gegebenenfalls durch geradkettiges oder verzweigtes Alkyl mit bis zu 8 Kohlenstoffatomen substituiert ist,

$R^2$ und $R^3$ gleich oder verschieden sind und für Wasserstoff oder für geradkettiges oder verzweigtes Alkyl mit bis zu 8 Kohlenstoffatomen stehen, oder für Benzyl stehen, oder für Cycloalkyl mit 3 bis 12 Kohlenstoffatomen stehen, das gegebenenfalls durch geradkettiges oder verzweigtes Alkyl mit bis zu 8 Kohlenstoffatomen substituiert ist, oder

$R^2$ und $R^3$ zusammen mit dem Stickstoffatom einen heterocyclischen Ring der Formel

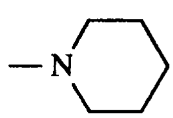

,

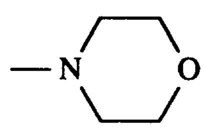

oder

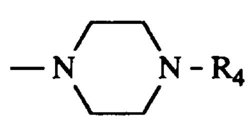

bilden, worin

$R^4$ geradkettiges oder verzweigtes Alkyl oder Alkenyl mit jeweils bis zu 8 Kohlenstoffatomen bedeutet, die gegegebenenfalls durch Phenyl substituiert sind oder Phenyl bedeutet, das gegebenenfalls durch Halogen oder Trifluormethyl substituiert ist,

gegebenenfalls in einer isomeren Form und deren Salze.

**2.** Chinolylmethoxyphenyl-essigsäureamide nach Anspruch 1, worin

A, B, D, E, G, L und M unabhängig voneinander für Wasserstoff, Hydroxy, Fluor, Chlor, Brom, Carboxy, Nitro, Trifluormethyl, Trifluormethoxy oder für geradkettiges oder verzweigtes Alkyl oder Alkoxy mit jeweils bis zu 6 Kohlenstoffatomen stehen, oder für Phenyl stehen, das gegebenenfalls durch Fluor, Chlor, Brom, Hydroxy, Nitro oder Cyano substituiert ist,

$R^1$ für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 6 Kohlenstoffatomen substituiert sind,

$R^2$ und $R^3$ gleich oder verschieden sind und für Wasserstoff oder für geradkettiges oder verzweigtes Alkyl mit bis zu 6 Kohlenstoffatomen stehen, oder für Benzyl stehen, oder für Cyclopropyl, Cyclobutyl, Cyclopentyl, Cyclohexyl, Cycloheptyl oder Cyclooctyl stehen, die gegebenenfalls durch geradkettiges oder verzweigtes Alkyl mit bis zu 6 Kohlenstoffatomen substituiert sind, oder:

$R^2$ und $R^3$ zusammen mit dem Stickstoffatom einen heterocyclischen Ring der Formel

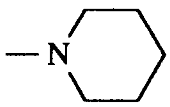

oder

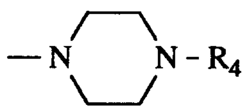

bilden, worin

$R^4$ geradkettiges oder verzweigtes Alkyl oder Alkenyl mit jeweils bis zu 6 Kohlenstoffatomen bedeutet, die gegebenenfalls durch Phenyl substituiert sind oder Phenyl bedeutet, das gegebenenfalls durch Fluor, Chlor oder Trifluormethyl substituiert ist,

gegebenenfalls in einer isomeren Form und deren Salze.

**3.** Chinolylmethoxyphenyl-essigsäureamide nach Anspruch 1, wobei

A, B, D, E, G, L und M unabhängig voneinander für Wasserstoff, Hydroxy, Fluor, Chlor, Brom oder geradkettiges oder verzweigtes Alkyl mit bis zu 4 Kohlenstoffatomen stehen,

$R^1$ für Cyclopropyl, Cyclopentyl, Cyclohexyl oder Cycloheptyl steht, die gegebenenfalls durch geradkettiges oder verzweigtes Alkyl mit bis zu 4 Kohlenstoffatomen substituiert sind,

$R^2$ und $R^3$ gleich oder verschieden sind und für Wasserstoff oder für geradkettiges oder verzweigtes Alkyl mit bis zu 4 Kohlenstoffatomen stehen, oder für Benzyl stehen, oder für Cyclopropyl, Cyclopentyl, Cyclohexyl oder Cycloheptyl stehen, die gegebenenfalls durch geradkettiges oder verzweigtes Alkyl mit bis zu 4

Kohlenstoffatomen substituiert sind, oder

$R^2$ und $R^3$ zusammen mit dem Stickstoffatom einen heterocyclischen Ring der Formel

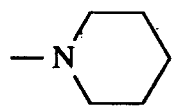

oder

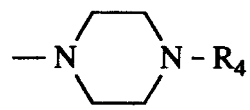

bilden, worin

$R^4$ geradkettiges oder verzweigtes Alkyl oder Alkenyl mit jeweils bis zu 4 Kohlenstoffatomen bedeutet, die gegebenenfalls durch Phenyl substituiert sind oder Phenyl bedeutet, das gegebenenfalls durch Fluor, Chlor oder Trifluormethyl substituiert ist,

gegebenenfalls in einer isomeren Form und deren Salze.

**4.** Chinolylmethoxyphenyl-essigsäureamide nach Anspruch 1, wobei A, B, D, E und G für Wasserstoff stehen.

**5.** Chinolylmethoxyphenyl-essigsäureamide nach Anspruch 1 zur therapeutischen Behandlung.

**6.** Verfahren zur Herstellung von Chinolylmethoxyphenyl-essigsäureamiden nach Anspruch 1, dadurch gekennzeichnet, daß man

[A] Säuren der allgemeinen Formel (II)

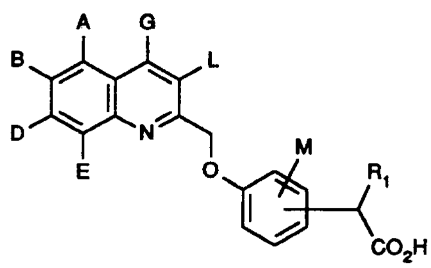

(II),

in welcher

A, B, D, E, G, L, M und $R^1$ die in Anspruch 1 angegebene Bedeutung haben,

nach Aktivierung der Carbonsäurefunktion, beispielsweise über die Säurehalogenide-, Anhydride oder Imidazolide, in organischen Lösemitteln mit Aminen der allgemeinen Formel (III)

$HNR^{2'}R^{3'}$ (III),

in welcher

$R^{2'}$ und $R^{3'}$ die in Anspruch 1 angegebene Bedeutung von $R^2$ und $R^3$ haben, aber nicht gleichzeitig für Wasserstoff stehen,

in inerten Lösemitteln, gegebenenfalls in Anwesenheit einer Base amidiert, oder

mit Ammoniak/Ammoniumchlorid umsetzt, oder

[B] Cyano-Verbindungen der allgemeinen Formel (IV)

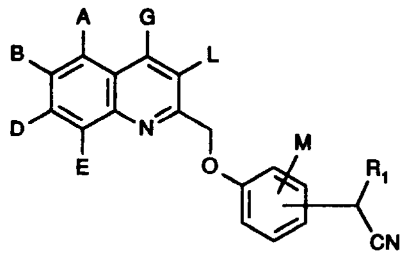

(IV),

in welcher
A, B, D, E, G, L, M und $R^1$ die in Anspruch 1 angegebene Bedeutung haben,
mit Säuren, vorzugsweise Salzsäure, umsetzt,
und im Fall, daß $R^2$ und/oder $R^3$ nicht Wasserstoff bedeuten,
gegebenenfalls auch eine Alkylierung der Aminogruppe anschließt,
und gegebenenfalls die Substituenten A, B, D, E, G, L und M nach üblichen Methoden variiert.

**7.** Arzneimittel enthaltend mindestens ein Chinolylmethoxyphenyl-essigsäureamid nach Anspruch 1.

**8.** Verfahren zur Herstellung von Arzneimitteln nach Anspruch 7, dadurch gekennzeichnet, daß man die Chinolylmethoxyphenyl-essigsäureamide gegebenenfalls mit Hilfe von üblichen Hilfs- und Trägerstoffen in eine geeignete Applikationsform bringt.

**9.** Verwendung von Chinolylmethoxyphenyl-essigsäureamiden nach Anspruch 1 zur Herstellung von Arzneimitteln.

**10.** Verwendung von Chinolylmethoxyphenyl-essigsäureamiden nach Anspruch 1 zur Herstellung von Leukotriensynthesehemmern.

Europäisches Patentamt

# EUROPÄISCHER RECHERCHENBERICHT

Nummer der Anmeldung

EP 92 11 9776

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 414 076 (BAYER AG)<br>* Seite 2 - Seite 4, Zeile 5 *<br>* Seite 7, Zeile 46 - Zeile 57 *<br>--- | 1,7 | C07D215/14<br>A61K31/47 |
| A,D | EP-A-0 399 291 (BAYER AG)<br>* Seite 2 - Seite 4, Zeile 26 *<br>* Seite 10, Zeile 23 - Zeile 31 *<br>--- | 1,7 | |
| A,D | EP-A-0 344 519 (BAYER AG)<br>* Seite 2 - Seite 8, Zeile 25 *<br>* Seite 16, Zeile 4 - Zeile 15 *<br>----- | 1,7 | |

RECHERCHIERTE SACHGEBIETE (Int. Cl.5 )

C07D

Der vorliegende Recherchenbericht wurde für alle Patentansprüche erstellt

| Recherchenort | Abschlußdatum der Recherche | Prüfer |
|---|---|---|
| BERLIN | 10 MAERZ 1993 | KYRIAKAKOU G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument